# EUROPEAN PATENT APPLICATION

(11) **EP 4 640 695 A1**
(43) Date of publication of application: **29.10.2025**
(21) Application number: 23906116.1
(22) Date of filing: 22.12.2023
(51) Int. Cl.: C07K 14/105, C12N 15/00, A61K 39/12

(54) **RECOMBINANT POLIOVIRUS-LIKE PARTICLE, PREPARATION METHOD THEREFOR, AND USE**

(30) Priority: 23.12.2022 CN 202211661333
(71) Applicant: Cansino Biologics Inc., Tianjin 300457 (CN)
(72) Inventor: YAN, Qiaoling, Tianjin 300457 (CN); SUN, Zhongyang, Tianjin 300457 (CN); LI, Hongyan, Tianjin 300457 (CN); ZHAO, Xiaoxue, Tianjin 300457 (CN); HAN, Qiqi, Tianjin 300457 (CN); YUAN, Hui, Tianjin 300457 (CN); ZHOU, Jin, Tianjin 300457 (CN); JI, Yuanyuan, Tianjin 300457 (CN); ZHAO, Huan, Tianjin 300457 (CN); MA, Liqiao, Tianjin 300457 (CN); ZHU, Tao, Tianjin 300457 (CN)
(74) Representative: Finnegan Europe LLP
(86) International application number: PCT/CN2023/141145
(87) International publication number: WO 2024/131961

(57) **Abstract**

Provided are a recombinant poliovirus-like particle (PVLP), a preparation method therefor, and a use. The mutation design of the recombinant poliovirus-like particle is based on a structural protein mutation combination, which not only enhances the thermal stability of the PVLP, but also improves the immunogenicity of the PVLP.

## Description

### CROSS-REFERENCE

The present disclosure claims priority to Chinese Patent Application No. 202211661333X filed on Dec. 23, 2022 and entitled "RECOMBINANT POLIOVIRUS-LIKE PARTICLE, PREPARATION METHOD THEREFOR, AND USE", which is incorporated herein by reference in its entirety as part of the present application.

### TECHNICAL FIELD

The present disclosure relates to the technical field of vaccines, and particularly relates to a recombinant poliovirus like particle, a preparation method therefor, and use thereof.

### BACKGROUND

Poliomyelitis, also known as infantile paralysis, is an acute enteric infectious disease primarily characterized by limb paralysis, caused by poliovirus infection and predominantly affecting children under five years of age. Currently, there is no effective treatment, and prevention can only be achieved through vaccination. *Poliovirus* (PV), belonging to the genus *Enterovirus* of the family *Picornaviridae*, possesses a single-stranded positive-sense RNA genome about 7.5 kb in length. The genome is divided into three parts: a 5' non-coding region, a polyprotein-coding region, and a 3' non-coding region. The polyprotein-coding region is directly translated into a polyprotein precursor upon infection of host cells, which is divided into three regions P1, P2, and P3. The P1 region is cleaved by its self-coded 3CD protease into three structural subunits VP0, VP1, and VP3. These three structural subunits are assembled into a viral structural protomer. Five protein protomers are assembled into a pentamer, and 12 pentamers are further assembled into a symmetric icosahedron. The major antigenic epitopes of the poliovirus are located on its structural proteins. Therefore, virus like particles (VLPs) can also stimulate the generation of an effective protective immune response and are promising vaccine candidates.

However, due to the structural conformational instability of *poliovirus* like particles (PVLPs), the poliovirus like particles are highly prone to transition from protective antigenic conformations, namely the D or N antigenic conformation, to non-protective antigenic conformations, namely the C or H conformation. To stabilize PVLPs, research groups from the University of Leeds, UK, and the NIBSC successively performed heat passaging, screening, and enrichment of viral variants bearing thermal stabilizing mutations. Through deep sequencing, they identified a set of mutation sites related to thermal stability on the structural proteins. However, these studies focused solely on thermal stability, i.e., screening for the optimal combination of mutation sites based on the temperature (Tm value) at which 50% of the D antigen transitions to the C antigen, without sufficient evaluation of the immunogenicity of the resulting PVLPs.

### SUMMARY

Through structural analysis of mutation sites of poliovirus type I, the present disclosure focuses on optimizing the mutation sites on VP1, and evaluates and screens the most effective mutation combination from the aspects of actual storage stability and immunogenicity to obtain at least 3 mutants that simultaneously address challenges in thermal stability and immunogenicity. Details are as follows.

In a first aspect of the present disclosure, provided is a recombinant *poliovirus* like particle (PVLP), wherein the PVLP is derived from: (i) strain Sabin 1 of poliovirus type I; (ii) strain MEF-1 of poliovirus type II; and/or (iii) strain Sabin 3 of poliovirus type III.

Preferably, the recombinant poliovirus like particle comprises three structural proteins VP0, VP1, and VP3, wherein VP0 can be cleaved into two subunits VP2 and VP4, and VP4 is located on the inner side of a capsid and connected to a virus core.

Preferably, the PVLP is derived from poliovirus type I, and a structural protein of the PVLP comprises a mutation in at least one residue at a protomer interface, a VP2/VP3 interface, and/or a pentamer interface of the protein relative to a structural protein coded by the poliovirus type I.

More preferably, the structural protein of the PVLP further comprises a mutation in at least one residue at a binding pocket domain of the protein and/or an internal network of the protein.

Further preferably, the structural protein of the PVLP derived from poliovirus type I comprises a mutation in at least one residue of each of functional domains of a protomer interface, a VP2/VP3 interface, a pentamer interface, and an internal network of the protein.

The functional domains refer to the protomer interface of the protein, the VP2/VP3 interface of the protein, the pentamer interface of the protein, the binding pocket domain of the protein, the internal network of the protein, and the like.

More preferably, the PVLP is derived from poliovirus type I, and the structural protein of the PVLP has the following mutations relative to a structural protein coded by the poliovirus type I:
(i-1) at least a mutation at residue 3178 in the protomer interface of the protein;
(i-2) at least a mutation at residue 3119 in the VP2/VP3 interface of the protein;
(i-3) at least one mutation at residues 2025 or 2057 in the pentamer interface of the protein.

Optionally, the protein of the PVLP further has the following mutations:
(i-4) at least one mutation at residues 1196 or 1134 in the binding pocket domain of the protein; and/or
(i-5) at least a mutation at residue 4018 in the internal network of the protein.

Preferably, the PVLP is derived from poliovirus type I, and the protein of the PVLP has the following mutations:
(i-1) at least residue mutation Q3178L in the protomer interface of the protein;
(i-2) at least residue mutation L3119M in the VP2/VP3 interface of the protein;
(i-3) at least one of residue mutations T2025A or D2057E in the pentamer interface of the protein.

Optionally, the protein of the PVLP further has the following mutations:
(i-4) at least one of residue mutations V1196L or F1134L in the pocket domain of the protein; and/or
(i-5) at least residue mutation R4018G in the internal network of the protein.

More preferably, the mutation comprises an insertion, a deletion, and/or a substitution.

More preferably, the PVLP is derived from poliovirus type I, and the protein of the PVLP has the following mutations:
i-a) T2025A, D2057E, L3119M, Q3178L, and R4018G; and
i-b) one or more of residue mutations H1248P, F1134L, and V1196L.

More preferably, the PVLP is derived from poliovirus type I, and the protein of the PVLP has the following mutations:
i-A) T2025A, D2057E, L3119M, Q3178L, H1248P, F1134L, V1196L, and R4018G; or
i-B) T2025A, D2057E, L3119M, Q3178L, F1134L, V1196L, and R4018G; or
i-C) T2025A, D2057E, L3119M, Q3178L, H1248P, V1196L, and R4018G; or
i-D) T2025A, D2057E, L3119M, Q3178L, H1248P, F1134L, and R4018G; or
i-E) T2025A, D2057E, L3119M, Q3178L, V1196L, and R4018G; or
i-F) T2025A, D2057E, L3119M, Q3178L, H1248P, and R4018G; or
i-G) T2025A, D2057E, L3119M, Q3178L, and H1248P; or
i-H) T2025A, D2057E, L3119M, Q3178L, F1134L, and R4018G; or
i-I) T2025A, D2057E, L3119M, Q3178L, and R4018G; or
i-J) T2025A, D2057E, L3119M, Q3178L, and R4018G.

More preferably, the PVLP is derived from poliovirus type I, and residue 1248 is not mutated in the protein of the PVLP; further preferably, the mutation comprises an insertion, a deletion, and/or a substitution.

The amino acid sequence of a P1 protein of the strain sabin1 of poliovirus type I is set forth in SEQ ID NO: 2; the amino acid sequence of a VP1 protein of the strain sabin1 of poliovirus type I is set forth in SEQ ID NO: 3; the amino acid sequence of a VP0 protein of the strain sabin1 of poliovirus type I is set forth in SEQ ID NO: 4; the amino acid sequence of a VP3 protein of the strain sabin1 of poliovirus type I is set forth in SEQ ID NO: 5; the amino acid sequence of a VP2 protein of the strain sabin1 of poliovirus type I is set forth in SEQ ID NO: 6. Preferably, the PVLP is derived from poliovirus type II, and the protein of the PVLP has the following mutations:
(ii-1) at least a mutation at residue 3178 in the protomer interface of the protein;
(ii-2) at least a mutation at residue 1041 in the pentamer interface of the protein; and/or
(ii-3) at least a mutation at residue 1159 in the pocket domain of the protein.

More preferably, the mutation comprises an insertion, a deletion, and/or a substitution.

More preferably, the PVLP is derived from poliovirus type II, and the protein of the PVLP has the following mutations:
(ii-1) at least residue mutation Q3178L in the protomer interface;
(ii-2) at least residue mutation T1041I in the pentamer interface; and/or
(ii-3) at least residue mutation Y1159F in the pocket domain.

The amino acid sequence of a P1 protein of the strain MEF-1 of poliovirus type II is set forth in SEQ ID NO: 7; the amino acid sequence of a VP1 protein of the strain MEF-1 of poliovirus type II is set forth in SEQ ID NO: 8; the amino acid sequence of a VP0 protein of the strain MEF-1 of poliovirus type II is set forth in SEQ ID NO: 9; the amino acid sequence of a VP3 protein of the strain MEF-1 of poliovirus type II is set forth in SEQ ID NO: 10; the amino acid sequence of a VP2 protein of the strain MEF-1 of poliovirus type II is set forth in SEQ ID NO: 11. Preferably, the PVLP is derived from poliovirus type III, and the protein of the PVLP has the following mutations:
(iii-1) at least one mutation at residues 2215 or 3091 in the protomer interface of the protein;
(iii-2) at least one mutation at residues 2018 or 3085 in the pentamer interface of the protein;
(iii-3) at least a mutation at residue 1132 in the pocket domain of the protein;
(iii-4) at least a mutation at residue 2241 in the VP2/VP3 interface of the protein; and/or
(iii-5) at least a mutation at residue 3019 in the internal network of the protein.

More preferably, the mutation comprises an insertion, a deletion, and/or a substitution.

More preferably, the PVLP is derived from poliovirus type III, and the protein of the PVLP has the following mutations:
(iii-1) at least one of residue mutations L2215M or F3091S in the protomer interface of the protein;
(iii-2) at least one of residue mutations L2018I or L3085F in the pentamer interface of the protein;
(iii-3) at least residue mutation F1132L in the pocket domain of the protein;
(iii-4) at least residue mutation D2241E in the VP2/VP3 interface of the protein; and/or
(iii-5) at least residue mutation H3019Y in the internal network of the protein.

The amino acid sequence of a P1 protein of the strain sabin3 of poliovirus type III is set forth in SEQ ID NO: 12;
the amino acid sequence of a VP1 protein of the strain sabin3 of poliovirus type III is set forth in SEQ ID NO: 13;
the amino acid sequence of a VP0 protein of the strain sabin3 of poliovirus type III is set forth in SEQ ID NO: 14;
the amino acid sequence of a VP3 protein of the strain sabin3 of poliovirus type III is set forth in SEQ ID NO: 15;
the amino acid sequence of a VP2 protein of the strain sabin3 of poliovirus type III is set forth in SEQ ID NO: 16.

In the numbering of the mutation sites, 1, 2, and 3 of position 1 correspond to structural proteins VP1, VP2, and VP3, respectively, and positions 2-4 of the numbering correspond to residue sites in the protein. For example, 2025 corresponds to the amino acid residue at position 25 on the VP2 structural protein, and T2025A corresponds to the amino acid residue at position 25 on the VP2 structural protein mutated from "T" to "A"; L3119M corresponds to the amino acid residue at position 119 on the VP3 structural protein mutated from "L" to "M"; V1196L corresponds to the amino acid residue at position 196 on the VP1 structural protein mutated from "V" to "L", and so on.

In the present disclosure, 2025 has the same meaning as the amino acid residue at position 25 on the VP2 structural protein, similarly, 1196 has the same meaning as the amino acid residue at position 196 on the VP1 structural protein, and so on.

In a second aspect of the present disclosure, provided is a gene encoding the recombinant poliovirus like particle described above.

Preferably, the gene comprises sequences VP1, VP0, and VP3 encoding proteins VP1, VP0, and VP3, wherein the VP1 protein has a mutation defined as any one of the above.

Preferably, the gene comprises a DNA or an RNA.

In a third aspect of the present disclosure, provided is a method for preparing the recombinant poliovirus like particle described above, wherein the preparation method comprises amplification to obtain the gene encoding the recombinant poliovirus like particle described above and construction of a vector carrying the gene described above.

Preferably, the preparation method comprises linking in tandem of any two structural protein genes of structural proteins VP1, VP0, and VP3, inserting them into a first expression cassette of the vector, and inserting another gene into a second expression cassette of the vector, thereby obtaining an expression vector capable of expressing different mutants of the poliovirus like particle.

More preferably, the two structural genes are linked in tandem via P2A.

In one specific embodiment, the amino acid sequence of P2A is set forth in SEQ ID NO: 1.

The P2A sequence is a nucleic acid sequence encoding an amino acid comprising D-X-E-X-N-P-G-P.

More preferably, the first expression cassette and the second expression cassette may be on the same expression vector or on different expression vectors. Further preferably, the first expression cassette and the second expression cassette are both on the same vector.

Preferably, a host cell of the vector comprises a mammalian cell (e.g., an HEK293 cell or a CHO cell), a yeast cell, or an insect cell (e.g., *Spodoptera frugiperda* cell Sf9 or *Trichoplusia ni* cell Tn); preferably, the host cell is *Spodoptera frugiperda* cell Sf9.

Preferably, the expression vector is a recombinant baculovirus, and the preparation method is to co-transfect shuttle plasmids inserted with different mutants of poliovirus like particles and a baculovirus genome in a *flash* BAC system into sf9 insect cells to obtain a recombinant baculoviruse capable of expressing different mutants of the poliovirus like particle.

Preferably, the method for preparing a shuttle plasmid comprises inserting genes of three structural proteins VP1, VP0, and VP3 into a pBacPAK8-Duet shuttle plasmid, wherein two structural genes are linked in tandem via IntP2A and then inserted into a first expression cassette of a pBacPAK8-Duet plasmid, and another gene is inserted into a second expression cassette of the pBacPAK8-Duet plasmid, thereby obtaining a shuttle plasmid capable of expressing different mutants of the poliovirus like particle.

Further preferably, the tandem structural gene comprises a combination of VP1 and VP0, a combination of VP0 and VP3, or a combination of VP1 and VP3. In one specific embodiment, the tandem structural gene comprises a combination of VP0 and VP3.

In a fourth aspect of the present disclosure, provided is a vector system carrying any one of the genes described above.

Preferably, the genes include genes VP1, VP0, and VP3.

More preferably, two structural genes of genes VP1, VP0, and VP3 are linked in tandem and then inserted into an expression cassette of a first plasmid, and another gene is inserted into an expression cassette of a second plasmid, thereby obtaining a transfer vector capable of expressing different mutants of the poliovirus like particle.

More preferably, the two structural genes are linked in tandem via int-P2A.

In one specific embodiment, the amino acid sequence of Int (Intein) is set forth in SEQ ID NO: 1.

The P2A sequence is a nucleic acid sequence encoding an amino acid comprising D-X-E-X-N-P-G-P.

More preferably, the vector system comprises a first plasmid and a second plasmid, and the first plasmid and the second plasmid may be identical or different. Further preferably, the first plasmid and the second plasmid are identical, and the plasmid is a pBacPAK8-Duet plasmid.

More preferably, the vector system comprises linking two structural genes in tandem via IntP2A followed by insertion into an expression cassette of one pBacPAK8-Duet plasmid and inserting another gene into an expression cassette of a second pBacPAK8-Duet plasmid, thereby obtaining a transfer vector capable of expressing different mutants of the poliovirus like particle.

Further preferably, the tandem structural gene comprises a combination of VP1 and VP0, a combination of VP0 and VP3, or a combination of VP1 and VP3. In one specific embodiment, the tandem structural gene comprises a combination of VP0 and VP3.

In a fifth aspect of the present disclosure, provided is use of the recombinant poliovirus like particle described above in preparing a medicament for preventing or treating poliomyelitis, wherein preferably, the medicament is a vaccine.

In a sixth aspect of the present disclosure, provided is a vaccine comprising the recombinant poliovirus like particle described above.

Preferably, the vaccine comprises 1-300 DU/dose of the recombinant poliovirus like particle described above per unit dose; more preferably, a content of PVLP type I is 1-300 DU/dose; a content of PVLP type II is 1-150 DU/dose; a content of PVLP type II is 1-150 DU/dose. The content of PVLP may be any range or value within the above range, e.g., 1-300 DU/dose, 25-280 DU/dose, 20-150 DU/dose, 30-200 DU/dose, 30-270 DU/dose, 50 DU/dose, 75 DU/dose, 90 DU/dose, 120 DU/dose, 230 DU/dose, etc.

Preferably, the vaccine further comprises an adjuvant. More preferably, the adjuvant is any one or more of an oil adjuvant, an oil-in-water adjuvant, a water-in-oil adjuvant, a water-in-oil-in-water adjuvant, an aluminum salt, and a polysaccharide adjuvant.

More preferably, the adjuvant is selected from any one or more of four aluminum adjuvants: aluminum hydroxide, aluminum hydroxide with unsaturated phosphate, aluminum hydroxide with saturated phosphate, and aluminum phosphate.

More preferably, a content of the adjuvant is 0.01-0.8 mg/dose, preferably 0.05-0.5 mg/dose.

Preferably, the vaccine further comprises a pharmaceutically acceptable auxiliary material; more preferably, the auxiliary material comprises an osmotic pressure regulator and a surfactant.

More preferably, a dosage form of the vaccine is any one or more of a liquid formulation, a powder, an injection, and a tablet; further preferably, the vaccine is a subcutaneous injection, an intramuscular injection, or a microinjection.

In a seventh aspect of the present disclosure, provided is a method for preventing poliomyelitis, and the method comprises administering any one of the recombinant poliovirus like particles or the vaccines described above to an individual.

The present disclosure has the following beneficial effects:
The mutation design of the recombinant poliovirus like particles in the present disclosure is based on a mutation combination on the structural proteins. The constructed PVLP has good integrity and is similar to poliovirus like particles in size and structure.

The optimized PVLP thermostable mutants enhance the thermal stability of PVLP particles.

The present disclosure also improves the immunogenicity of PVLPs, enabling them to induce a high level of antibody immune response. The present disclosure provides a vaccine comprising a recombinant poliovirus like particle, and the vaccine is safe, effective, and of controlled quality.

### BRIEF DESCRIPTION OF THE DRAWINGS

FIG. 1 is a schematic diagram of key sites on the surface of poliovirus type I VP1 (legend: VP1 is shown in the center, VP2 in the upper left part, and VP3 in the lower left part. The lipid molecules in the hydrophobic pockets are displayed as spherical structures, and the neutralizing epitope (peptide segment 90-104) on the VP1 surface structure is highlighted. The mutation sites (F134, V196, and H248) are displayed as spherical structures. The rest of the structure is depicted in cartoon mode);
FIG. 2 shows the stability results for mutants of recombinant poliovirus like particles type I;
FIG. 3 shows the stability results for mutants of recombinant poliovirus like particles type II;
FIG. 4 shows the stability results for mutants of recombinant poliovirus like particles type III;
FIG. 5 is an electron micrograph of the unmutated SN1/0-VLP;
FIG. 6 is an electron micrograph of the mutated SN1/m3c-VLP;
FIG. 7 is an electron micrograph of the mutated SN1/m3b-VLP;
FIG. 8 is an electron micrograph of the mutated SN1/m3a-VLP;
FIG. 9 is an electron micrograph of the mutated SN1/m3-VLP;
FIG. 10 is an electron micrograph of the mutated SN1/m4-VLP;
FIG. 11 is an electron micrograph of the mutated SN1/m5a-VLP;
FIG. 12 is an electron micrograph of the mutated SN1/m5-VLP;
FIG. 13 is an electron micrograph of the mutated SN1/m6c-VLP;
FIG. 14 is an electron micrograph of the mutated SN1/m6b-VLP;
FIG. 15 is an electron micrograph of the mutated SN1/m6a-VLP;
FIG. 16 is an electron micrograph of the mutated SN1/m7c-VLP;
FIG. 17 is an electron micrograph of the mutated SN1/m7b-VLP;
FIG. 18 is an electron micrograph of the mutated SN1/m7a-VLP;
FIG. 19 is an electron micrograph of the mutated SN1/m8-VLP;
FIG. 20 is an electron micrograph of the unmutated MEF/0-VLP;
FIG. 21 is an electron micrograph of the mutated MEF/1c-VLP;
FIG. 22 is an electron micrograph of the mutated MEF/1b-VLP;
FIG. 23 is an electron micrograph of the mutated MEF/1a-VLP;
FIG. 24 is an electron micrograph of the mutated MEF/2c-VLP;
FIG. 25 is an electron micrograph of the mutated MEF/2b- VLP;
FIG. 26 is an electron micrograph of the mutated MEF/2a-VLP;
FIG. 27 is an electron micrograph of the mutated MEF/3-VLP;
FIG. 28 is an electron micrograph of the mutated MEF/3c-VLP;
FIG. 29 is an electron micrograph of the mutated MEF/3b-VLP;
FIG. 30 is an electron micrograph of the mutated MEF/3a-VLP;
FIG. 31 is an electron micrograph of the mutated MEF/4d-VLP;
FIG. 32 is an electron micrograph of the mutated MEF/4c-VLP;
FIG. 33 is an electron micrograph of the mutated MEF/4b-VLP;
FIG. 34 is an electron micrograph of the mutated MEF/4a-VLP;
FIG. 35 is an electron micrograph of the mutated MEF/4;
FIG. 36 is an electron micrograph of the mutated MEF/5;
FIG. 37 is an electron micrograph of the unmutated SN3/0-VLP;
FIG. 38 is an electron micrograph of the mutated SN3/7a-VLP;
FIG. 39 is an electron micrograph of the mutated SN3/7b-VLP;
FIG. 40 is an electron micrograph of the mutated SN3/7c-VLP;
FIG. 41 is an electron micrograph of the mutated SN3/7d-VLP;
FIG. 42 is an electron micrograph of the mutated SN3/7e-VLP;
FIG. 43 is an electron micrograph of the mutated SN3/7f-VLP;
FIG. 44 is an electron micrograph of the mutated SN3/7g-VLP;
FIG. 45 is an electron micrograph of the mutated SN3/7h-VLP;
FIG. 46 is an electron micrograph of the mutated SN3/8-VLP;
FIG. 47 is a schematic diagram showing the immunogenicity results for different mutants of recombinant poliovirus like particles type I;
FIG. 48 is a schematic diagram showing the immunogenicity results for different mutants of recombinant poliovirus like particles type II;
FIG. 49 is a schematic diagram showing the immunogenicity results for different mutants of recombinant poliovirus like particles type III;

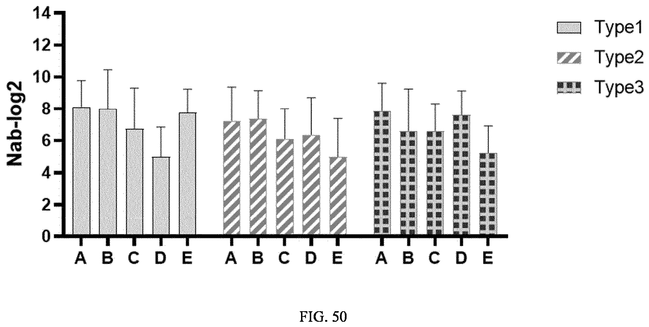

FIG. 50 is a schematic diagram showing the immunogenicity results for different mutants of recombinant poliovirus like particles types I-II-III.

### DETAILED DESCRIPTION

The technical solutions of the present disclosure will be clearly and completely described below with reference to the drawings of the present disclosure, and apparently, the described examples are only exemplary embodiments of the present disclosure instead of all embodiments of the present disclosure. Based on the examples of the present disclosure, all other examples obtained by those of ordinary skill in the art without creative work shall fall within the protection scope of the present disclosure.

### Example 1: Selection and Design of Mutation Sites for Poliovirus Antigens

In this experiment, the structural sequence of sabin1 was selected, and the number and combination form of candidate thermal stability-related mutation sites on VP1 were optimized. In this experiment, the insect cell-baculovirus system was selected for the expression and preparation of different VLP mutants. A total of 15 mutants were designed, and the specific mutation sites are shown in Table 1.

**Table 1. Design of thermostable mutants for poliovirus type I**

| No. | Sample code | Mutation site | | | | | | | |
|---|---|---|---|---|---|---|---|---|---|
| | | Pentamer interface | | Protomer interface | | Hydrophobic pocket | | Internal network | VP2/3 interface |
| | | T2025A | D2057E | Q3178L | H1248P | F1134L | V1196L | R4018G | L3119M |
| 1 | SN1/m8 | Y | Y | Y | Y | Y | Y | Y | Y |
| 2 | SN1/m7a | Y | Y | Y | - | Y | Y | Y | Y |
| 3 | SN1/m7b | Y | Y | Y | Y | - | Y | Y | Y |
| 4 | SN1/m7c | Y | Y | Y | Y | Y | - | Y | Y |
| 5 | SN1/m6a | Y | Y | Y | - | - | Y | Y | Y |
| 6 | SN1/m6b | Y | Y | Y | Y | - | - | Y | Y |
| 7 | SN1/m6c | Y | Y | Y | - | Y | - | Y | Y |
| 8 | SN1/m5 | Y | Y | Y | - | - | - | Y | Y |
| 9 | SN1/m5a | Y | Y | Y | Y | - | - | - | Y |
| 10 | SN1/m4 | Y | Y | Y | - | - | - | - | Y |
| 11 | SN1/m3 | Y | Y | Y | - | - | - | - | - |
| 12 | SN1/m3a | Y | Y | - | - | - | - | - | Y |
| 13 | SN1/m3b | Y | - | Y | - | - | - | - | Y |
| 14 | SN1/m3c | - | Y | Y | - | - | - | - | Y |
| 15 | SN1/0 | - | - | - | - | - | - | - | - |

| | | | | | | | | | |
|---|---|---|---|---|---|---|---|---|---|
| Note: Y indicates mutation; - indicates no mutation. | | | | | | | | | |

The selected mutation site combinations for poliovirus like particles type II and type III are shown in Table 2 and Table 3, respectively. Virus particles type II were derived from strain MEF-1 of poliovirus type II, and virus-like particles type III were derived from strain sabin 3 of poliovirus type III.

**Table 2. Design of thermostable mutants for poliovirus type II**

| No. | Sample code | Mutation site | | | | |
|---|---|---|---|---|---|---|
| | | Pocket structure | | Protomer interface | Pentamer interface | |
| | | F1143L | Y1159F | Q3178L | L3085F | T1041I |
| 1 | MEF/5 | Y | Y | Y | Y | Y |
| 2 | MEF/4 | - | Y | Y | Y | Y |
| 3 | MEF/4a | Y | - | Y | Y | Y |
| 4 | MEF/4b | Y | Y | - | Y | Y |
| 5 | MEF/4c | Y | Y | Y | - | Y |
| 6 | MEF/4d | Y | Y | Y | Y | - |
| 7 | MEF/3a | Y | - | Y | Y | - |
| 8 | MEF/3b | Y | - | Y | - | Y |
| 9 | MEF/3c | - | Y | Y | Y | - |
| 10 | MEF/3 | - | Y | Y | - | Y |
| 11 | MEF/2a | - | - | Y | - | Y |
| 12 | MEF2b | - | Y | - | - | Y |
| 13 | MEF/2c | - | Y | Y | - | - |
| 14 | MEF/1a | - | - | Y | - | - |
| 15 | MEF/1b | - | - | - | - | Y |
| 16 | MEF/1c | - | Y | - | - | - |
| 17 | MEF/0 | - | - | - | - | - |

| | | | | | | |
|---|---|---|---|---|---|---|
| Note: Y indicates mutation; - indicates no mutation. | | | | | | |

**Table 3. Design of thermostable mutants for poliovirus type III**

| Sample code | Mutation site | | | | | | | |
|---|---|---|---|---|---|---|---|---|
| | L2018I | L2215M | D2241E | H3019Y | L3085F | F3091S | A1054V | F1132L |
| SN3/0 | - | - | - | - | - | - | - | - |
| SN3/7a | - | Y | Y | Y | Y | Y | Y | Y |
| SN3/7b | Y | - | Y | Y | Y | Y | Y | Y |
| SN3/7c | Y | Y | - | Y | Y | Y | Y | Y |
| SN3/7d | Y | Y | Y | - | Y | Y | Y | Y |
| SN3/7e | Y | Y | Y | Y | - | Y | Y | Y |
| SN3/7f | Y | Y | Y | Y | Y | - | Y | Y |
| SN3/7g | Y | Y | Y | Y | Y | Y | - | Y |
| SN3/7h | Y | Y | Y | Y | Y | Y | Y | - |
| SN3/8 | Y | Y | Y | Y | Y | Y | Y | Y |

| | | | | | | | | |
|---|---|---|---|---|---|---|---|---|
| Note: Y indicates mutation; - indicates no mutation. | | | | | | | | |

### Example 2: Construction of Expression Vectors for Poliovirus Mutants

The VP1 genes of the wild-type SN1/0 and the mutant SN1/m8 containing 8 point mutation sites in Table 1 of Example 1, as well as the VP3-2A-VP0 tandem gene, were synthesized separately. The VP1 genes were inserted downstream of the P10 promoter in the pBacPAK8-Duet shuttle plasmid, while the VP3-2A-VP0 gene was inserted downstream of the Ppolh promoter in the same shuttle plasmid. This yielded shuttle plasmids pBacPAK8-SN1/0-VP1-VP3intP2AVP0 and pBacPAK8-SN1/m8-VP1-VP3intP2AVP0, i.e., shuttle plasmids expressing the wild-type virus-like particles and the virus-like particles containing 8 point mutation sites of strain sabin1 of poliovirus type I, respectively.

Then, pBacPAK8-SN1/m8-VP1-VP3intP2AVP0 was used as a template, and single-point mutations were performed by homologous recombination to construct shuttle plasmids expressing the virus-like particles containing other mutation sites in Table 1 of Example 1.

Similarly, shuttle plasmids for the non-mutant and mutation sites of poliovirus like particles type II in Table 2 of Example 1 and the non-mutant and mutation sites of poliovirus like particles type III in Table 3 of Example 1 were synthesized.

### Example 3: Preparation and Amplification of Recombinant Poliovirus Baculoviruses

The 15 shuttle plasmids containing different mutation combinations in Table 1 described in Example 2 were separately co-transfected into sf9 insect cells using a *flash* BAC system to obtain the first-generation baculoviruses of poliovirus like particles capable of expressing different mutation combinations. The first-generation baculoviruses were successively amplified for two generations in sf9 cells to obtain the third-generation viruses. The virus concentration of each generation was determined by the CCID50 method. The determination method was as follows: diluted insect cells (sf9-ET cells) were added to a 96-well plate at 100 µL/well. The baculoviruses to be tested were serially 10-fold diluted with culture medium to prepare virus dilutions ranging from 10⁻¹, 10⁻² to 10⁻⁹. The virus dilutions were added to the 96-well plate coated with sf9-ET cells at 100 µL/well, with 12 replicate wells set for each dilution. The plate was placed in a cell incubator for static culture at 27 °C±1 °C for 6-9 days. The cell infection state at each dilution factor was observed under a microscope and the number of positive infection wells was recorded. Finally, the Reed-Muench method was used for result calculation.

Similarly, according to Tables 2 and 3, recombinant baculoviruses of poliovirus like particles types II and III capable of expressing different mutation combinations were separately prepared and successively amplified for two generations to obtain the third-generation viruses.

### Example 4: Expression and Purification of Mutants of Recombinant Poliovirus Like Particles

The third-generation recombinant baculoviruses of the three serotypes obtained in Example 3 were separately used at an MOI of 1 to infect 250 mL of sf9 suspension cells (cell density of 4E+06 cells/mL), which were cultured at 27 °C±1 °C for 6-8 days using sf900 IIISFM. After the cell viability decreased to 20% or lower, the cell suspensions were collected and centrifuged at 5000 rpm for 30 min, and the supernatants were collected. The harvested supernatants were subjected to 10-fold ultrafiltration concentration using a 100 kD membrane, yielding a concentrated solution with a volume of 25-30 mL. Ultracentrifugation was performed using 15%-30% sucrose gradients. After 4 h of ultracentrifugation, 12 sucrose gradients (1 mL/gradient) were collected from top to bottom. Western blot assay was performed, and gradients containing the target product were collected according to the results.

### Example 5: Stability Comparison of Mutants of Recombinant Poliovirus Like Particles

Different mutants of poliovirus like particles type I obtained in Example 4 were aliquoted and then stored at 37 °C. Samples were taken at time points of 1 week, 2 weeks, 4 weeks, 6 weeks, and 8 weeks for detection of the effective antigen content, and the antigen content was compared with that at zero.

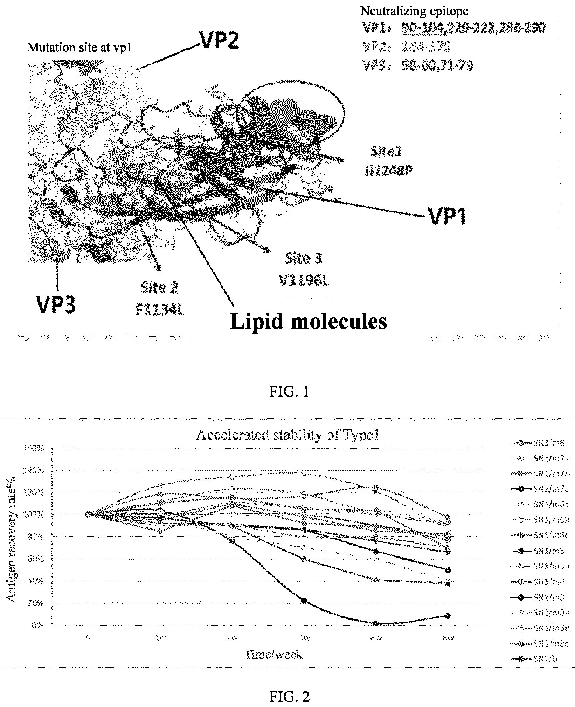

The detection results are shown in Table 4 and FIG. 2. The results show that the wild-type SN1/0 without point mutation and mutants m7c, m5, m3a, and m3 had poor stability, and m4, m5a, m6a, m6b, m7a, m7b, and m8 had the best stability.

**Table 4. Stability results for mutants of recombinant poliovirus like particles type I**

| Days | 0 | 1 week | 2 weeks | 4 weeks | 6 weeks | 8 weeks |
|---|---|---|---|---|---|---|
| SN1/m8 | 100% | 101% | 100% | 100% | 90% | 80% |
| SN1/m7a | 100% | 126% | 134% | 137% | 121% | 87% |
| SN1/m7b | 100% | 118% | 114% | 117% | 124% | 97% |
| SN1/m7c | 100% | 104% | 76% | 22% | 2% | 8% |
| SN1/m6a | 100% | 103% | 100% | 100% | 103% | 92% |
| SN1/m6b | 100% | 112% | 123% | 118% | 101% | 92% |
| SN1/m6c | 100% | 110% | 116% | 105% | 104% | 69% |
| SN1/m5 | 100% | 92% | 91% | 87% | 76% | 66% |
| SN1/m5a | 100% | 98% | 111% | 106% | 100% | 93% |
| SN1/m4 | 100% | 95% | 110% | 98% | 85% | 82% |
| SN1/m3 | 100% | 97% | 90% | 86% | 67% | 50% |
| SN1/m3a | 100% | 98% | 80% | 70% | 60% | 40% |
| SN1/m3b | 100% | 90% | 91% | 79% | 80% | 70% |
| SN1/m3c | 100% | 85% | 108% | 92% | 89% | 77% |
| SN1/0 | 100% | 97% | 89% | 60% | 41% | 38% |

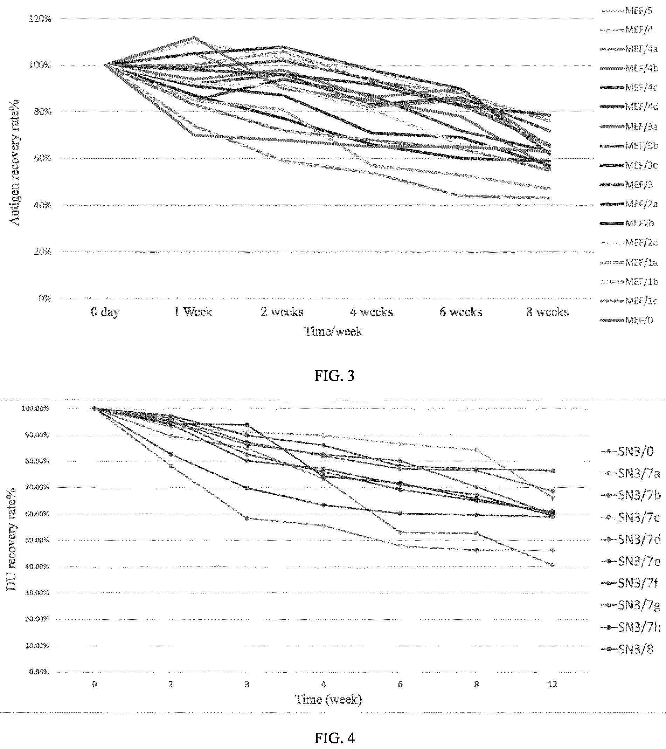

Different mutants of poliovirus like particles type II obtained in Example 4 were aliquoted and then stored at 37 °C. Samples were taken at time points of 1 week, 2 weeks, 4 weeks, 6 weeks, and 8 weeks for detection of the effective antigen content, and the antigen content was compared with that at zero. The results are shown in Table 5 below and FIG. 3.

**Table 5. Stability results for mutants of recombinant poliovirus like particles type II**

| Days | 0 day | 1 week | 2 weeks | 4 weeks | 6 weeks | 8 weeks |
|---|---|---|---|---|---|---|
| MEF/5 | 100% | 110% | 103% | 98% | 85% | 78% |
| MEF/4 | 100% | 100% | 106% | 93% | 88% | 76% |
| MEF/4a | 100% | 105% | 91% | 82% | 85% | 65% |
| MEF/4b | 100% | 112% | 90% | 85% | 78% | 56% |
| MEF/4c | 100% | 92% | 96% | 83% | 86% | 72% |
| MEF/4d | 100% | 85% | 94% | 87% | 72% | 63% |
| MEF/3a | 100% | 94% | 98% | 86% | 90% | 65% |
| MEF/3b | 100% | 99% | 102% | 94% | 83% | 66% |
| MEF/3c | 100% | 105% | 108% | 98% | 90% | 62% |
| MEF/3 | 100% | 98% | 96% | 92% | 83% | 78% |
| MEF/2a | 100% | 91% | 87% | 71% | 69% | 57% |
| MEF2b | 100% | 87% | 77% | 66% | 60% | 59% |
| MEF/2c | 100% | 92% | 91% | 81% | 66% | 63% |
| MEF/1a | 100% | 85% | 81% | 57% | 53% | 47% |
| MEF/1b | 100% | 74% | 59% | 54% | 44% | 43% |
| MEF/1c | 100% | 83% | 72% | 68% | 64% | 55% |
| MEF/0 | 100% | 70% | 68% | 65% | 65% | 63% |

The detection results are shown in FIG. 3. The results show that there were three trends in stability change, and mutants MEF/3, MEF/4, MEF/5, and MEF/4c had the best stability.

Different mutants of poliovirus like particles type III obtained in Example 4 were aliquoted and then stored at 37 °C. Samples were taken at time points of 1 week, 2 weeks, 4 weeks, 6 weeks, and 8 weeks for detection of the effective antigen content, and the antigen content was compared with that at zero. The results are shown in Table 6 below and FIG. 4.

**Table 6. Stability results for mutants of recombinant poliovirus like particles type III**

| Days | 0 | 1 week | 2 weeks | 4 weeks | 6 weeks | 8 weeks |
|---|---|---|---|---|---|---|
| SN3/0 | 100% | 58% | 56% | 48% | 46% | 46% |
| SN3/7a | 100% | 91% | 90% | 87% | 84% | 66% |
| SN3/7b | 100% | 87% | 82% | 77% | 76% | 69% |
| SN3/7c | 100% | 85% | 73% | 53% | 53% | 41% |
| SN3/7d | 100% | 70% | 63% | 60% | 55% | 54% |
| SN3/7e | 100% | 80% | 67% | 77% | 71% | 59% |
| SN3/7f | 100% | 83% | 76% | 69% | 65% | 61% |
| SN3/7g | 100% | 86% | 83% | 80% | 70% | 60% |
| SN3/7h | 100% | 94% | 74% | 72% | 66% | 61% |
| SN3/8 | 100% | 90% | 86% | 78% | 77% | 76% |

The detection results are shown in FIG. 4. The results show that the mutants had significantly improved stability relative to the unmutated protein, and especially in the first 8 weeks, mutant SN3/8 had the best stability.

### Example 6: Electron Microscopy Analysis of Mutants of Recombinant Poliovirus Like Particles

The purified virus-like particles of the three serotypes obtained in Example 4 were separately stained with uranyl acetate to prepare samples and observed under an electron microscope at 120 kV.

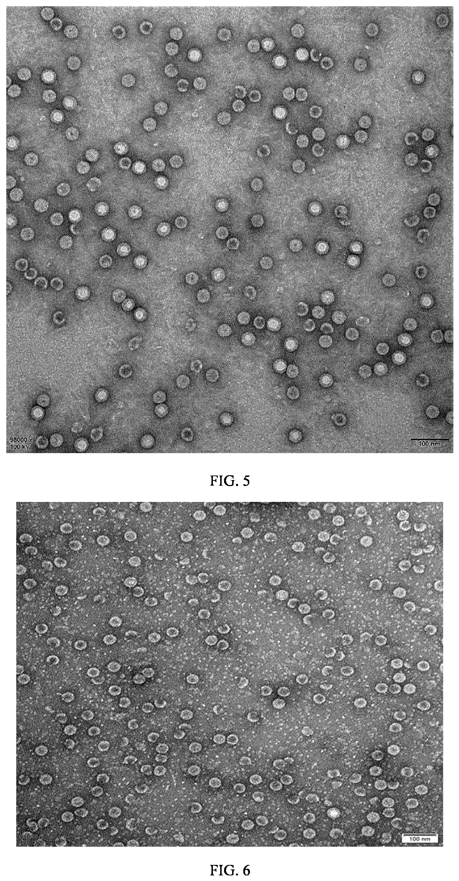

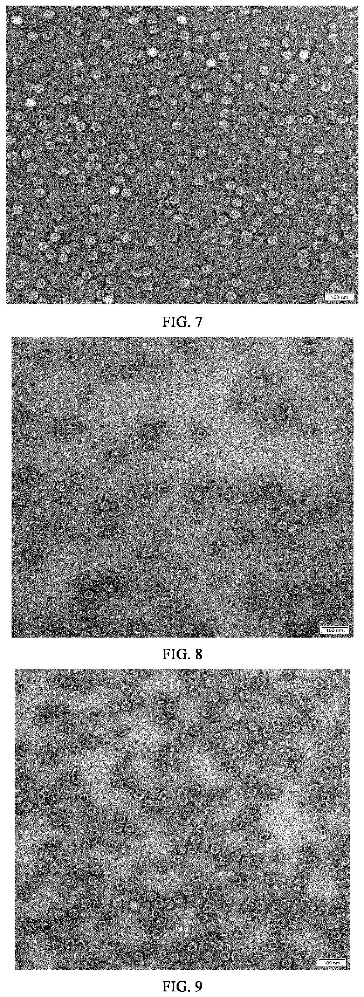

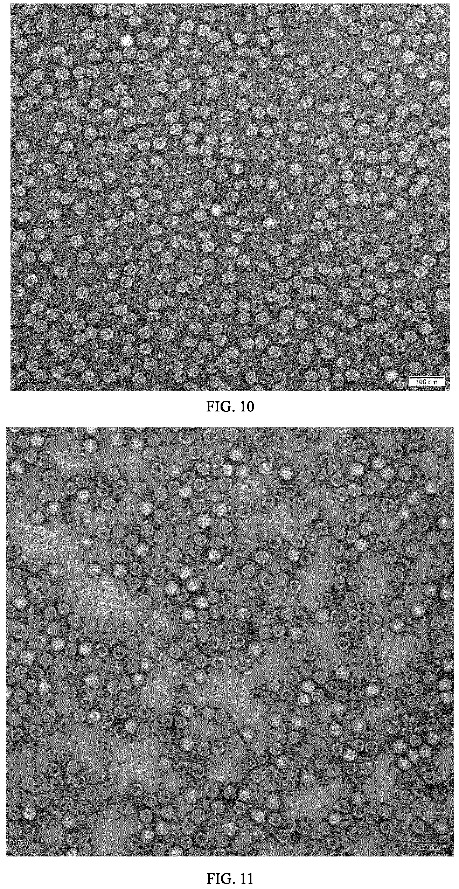

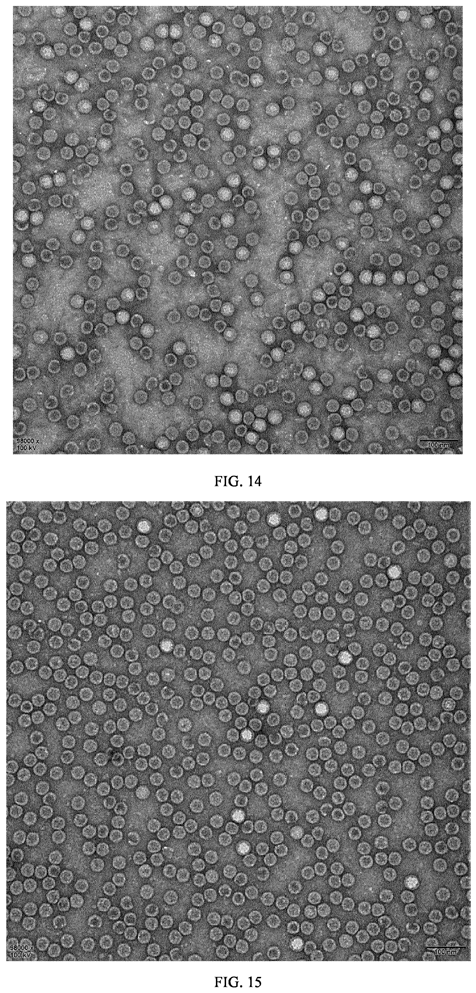

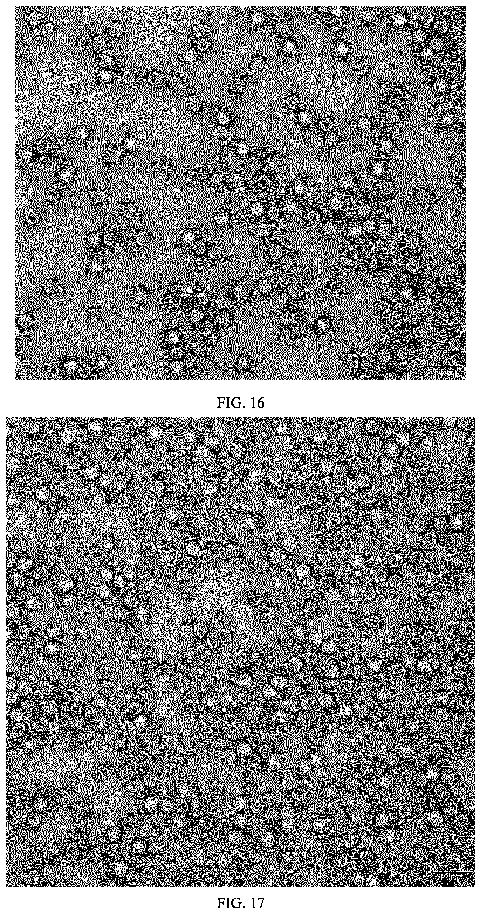

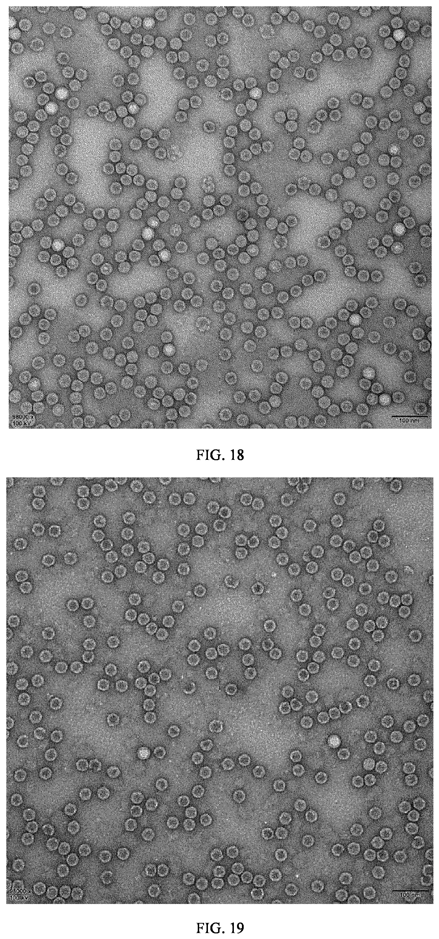

The electron micrographs 5-19 show that the virus-like particle spherical appearances of SN1/m3 (FIG. 6), SN1/m3a (FIG. 7), SN1/m3b (FIG. 8), SN1/m3c (FIG. 9), SN1/m5a (FIG. 11), SN1/m6b (FIG. 14), SN1/m7c (FIG. 16), SN1/m7b (FIG. 17), and SN1/m8 (FIG. 19) were similar to or slightly better than the virus-like particle spherical appearance of the non-mutant SN1/0 (FIG. 5), and there was a high portion of particles with an incomplete, irregular, and relatively randomly shaped virus-like particle spherical appearance.

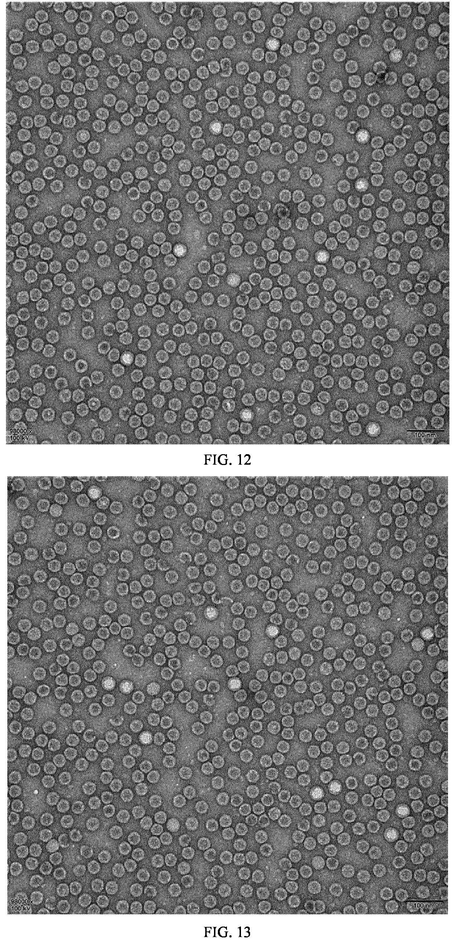

VLP particles of SN1/m4 (FIG. 10), SN1/m5 (FIG. 12), SN1/m6c (FIG. 13), SN1/m6a (FIG. 15), and SN1/m7a (FIG. 18) had a diameter of about 35 nm, were uniform in size, did not show VLP aggregation, and showed a relatively complete spherical appearance.

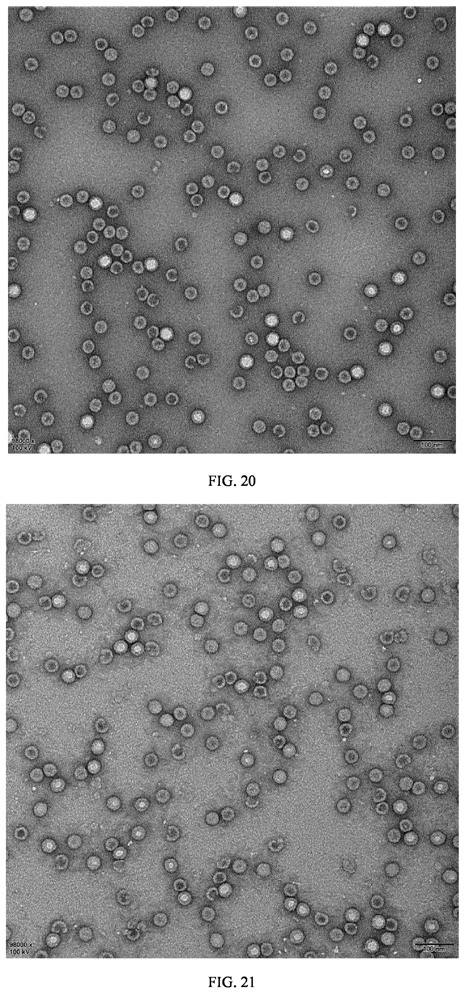

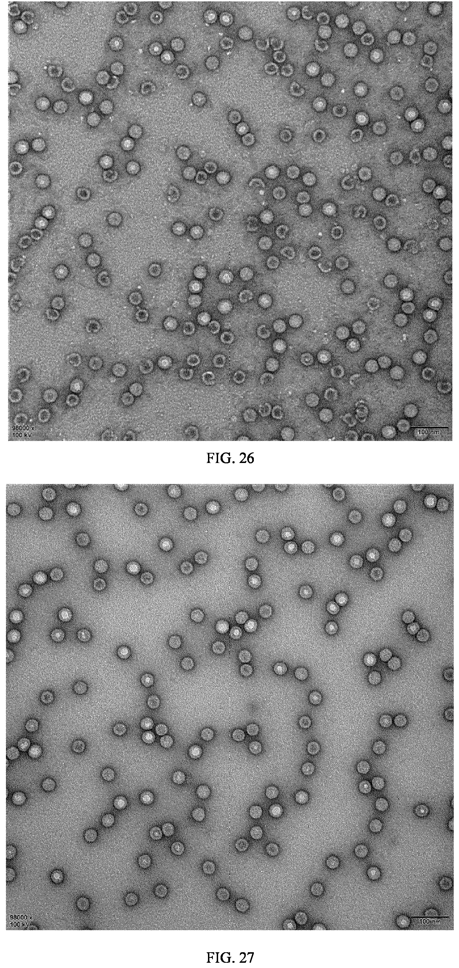

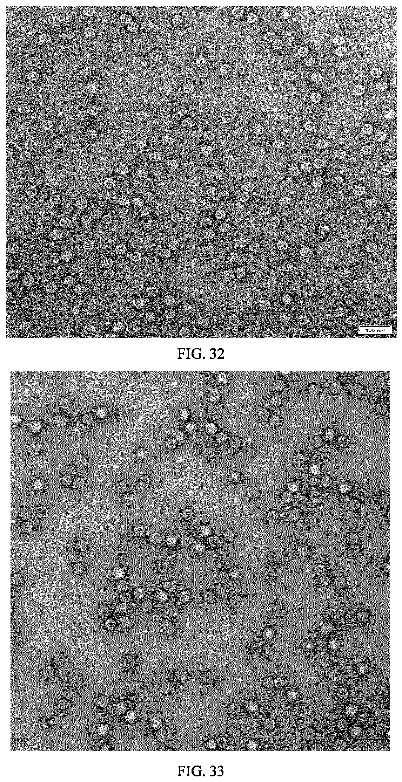

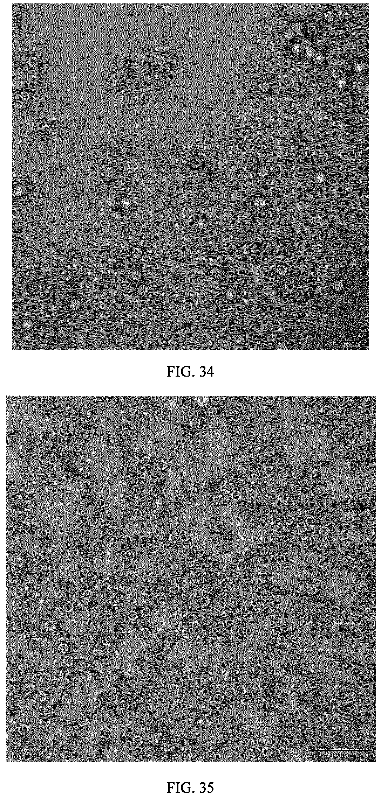

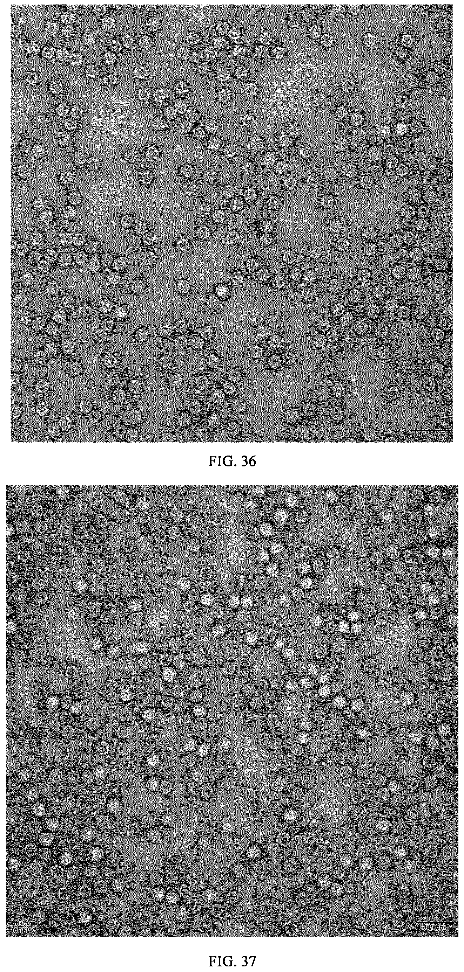

It can be seen from electron micrographs 20-36 that compared with the unmutated MEF/0 (FIG. 20), the mutated MEF/3 VLP particles (FIG. 27), MEF/4c VLP particles (FIG. 32), MEF/4 VLP particles (FIG. 35), and MEF/5 VLP particles (FIG. 36) had a diameter of about 37 nm, were uniform in size, did not show VLP aggregation, and showed a relatively complete spherical appearance.

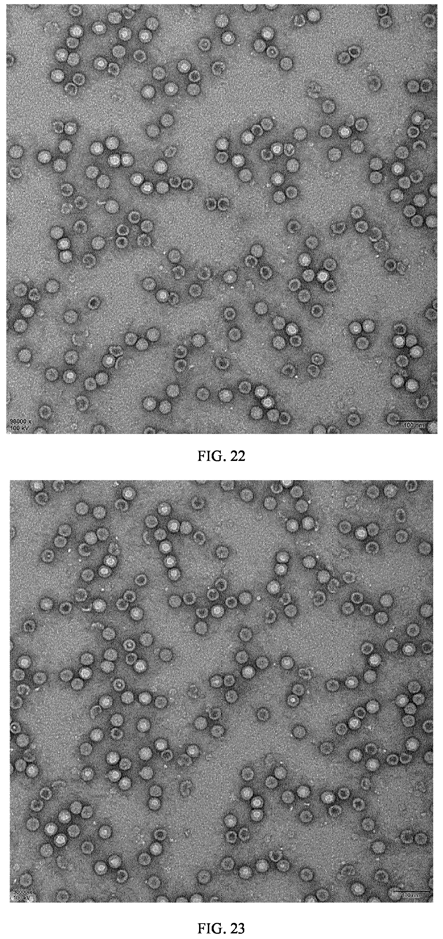

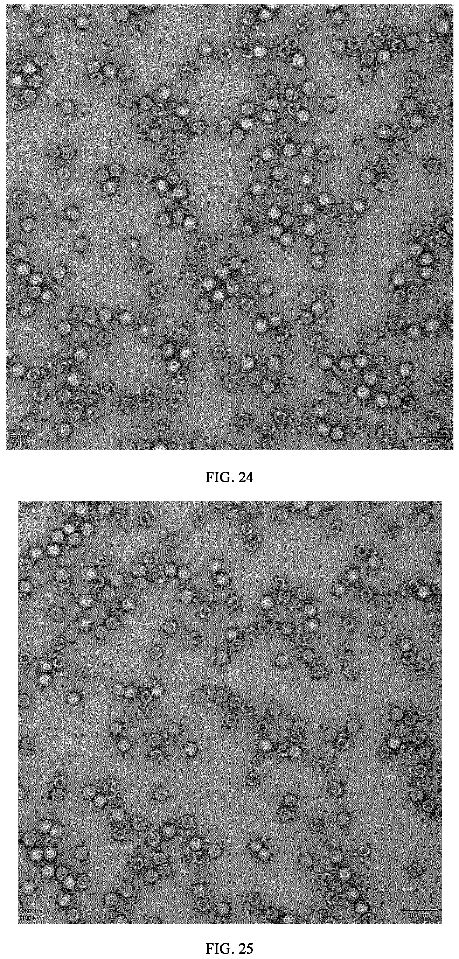

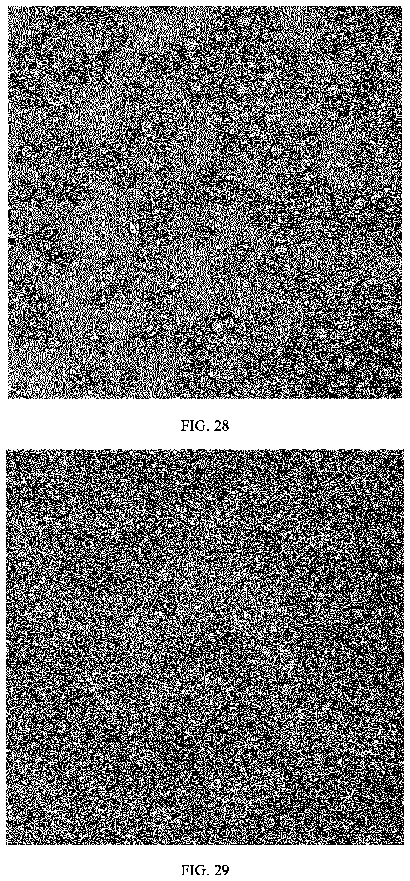

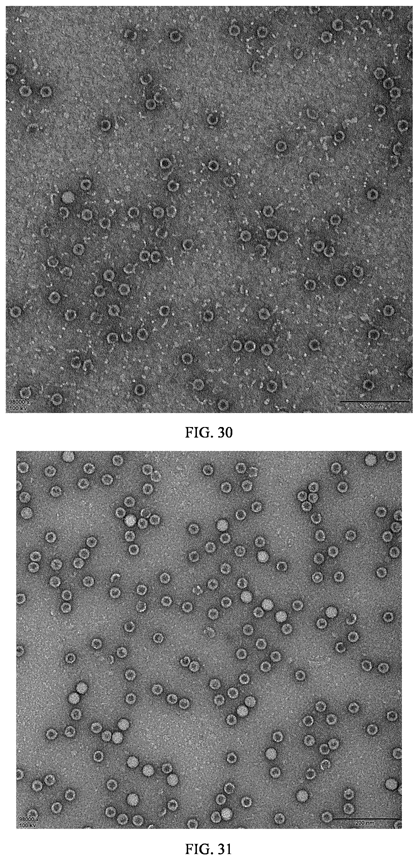

Secondly, it can be seen from the electron micrographs that MEF/2a (FIG. 26), MEF/2b (FIG. 25), MEF/2c (FIG. 24), MEF/1a (FIG. 23), MEF/1b (FIG. 22), MEF/1c (FIG. 21), MEF/3c (FIG. 28), MEF/3b (FIG. 29), MEF/3a (FIG. 30), MEF/4d (FIG. 31), MEF/4a (FIG. 34), and MEF/4b (FIG. 33) had a high portion of particles with an incomplete, irregular, and relatively randomly shaped virus-like particle spherical appearance.

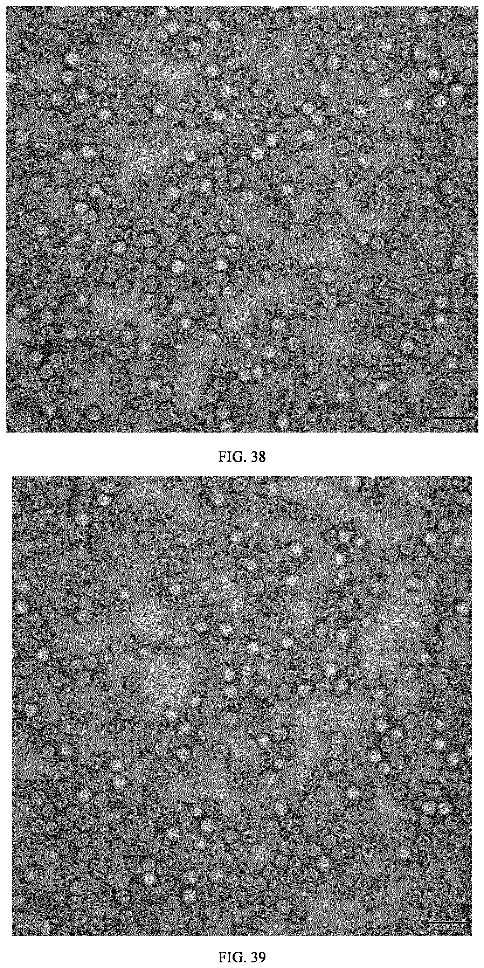

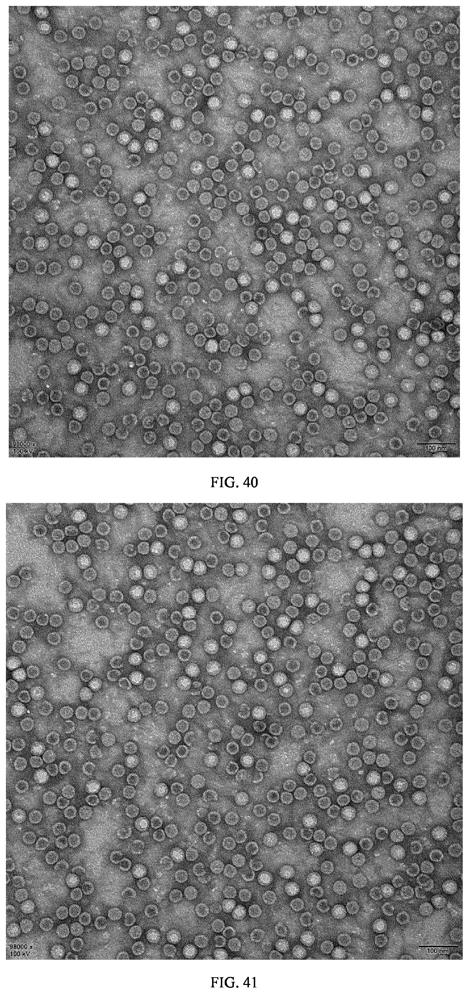

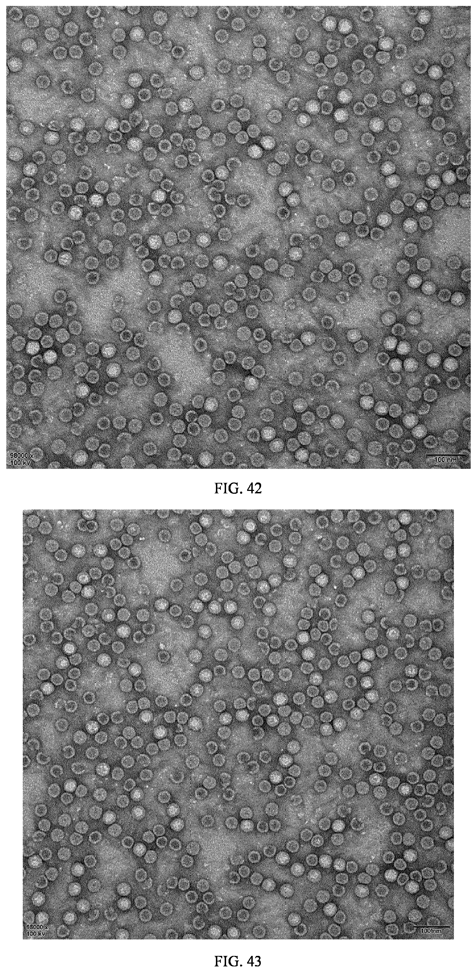

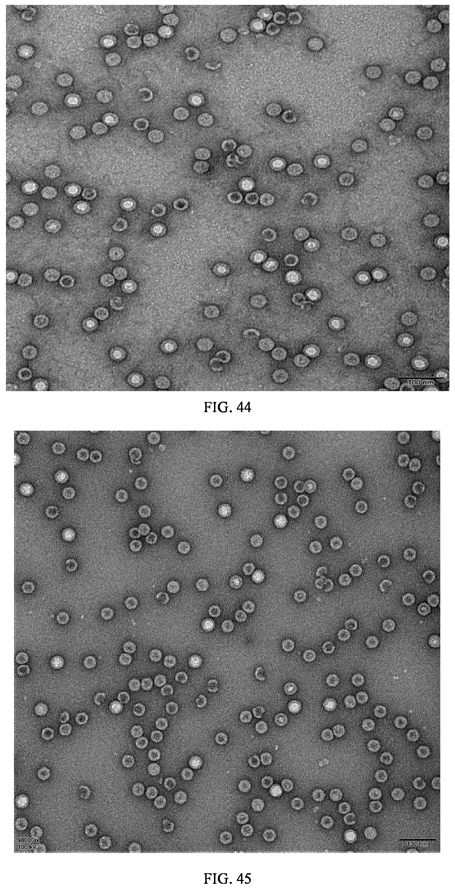

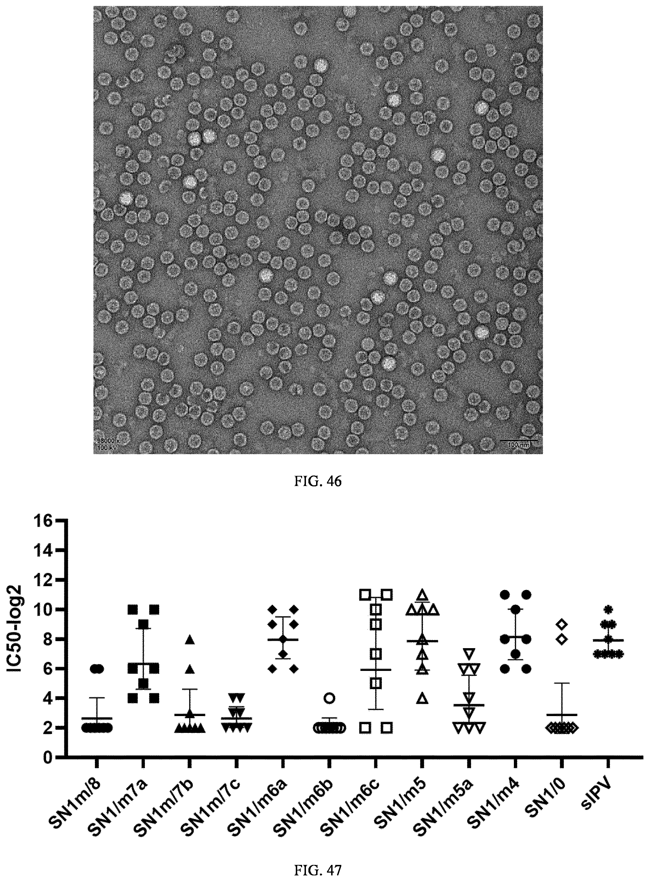

It can be seen from electron micrographs 37-46 that compared with the unmutated SN3/0 (FIG. 37), the mutated SN3/8 VLP particles (FIG. 46) had a particle diameter of about 34 nm, were uniform in size, did not show VLP aggregation, and showed a relatively complete spherical appearance. The roundness of VLPs of the mutated SN3/7a (FIG. 38), SN3/7b (FIG. 39), SN3/7c (FIG. 40), SN3/7d (FIG. 41), SN3/7e (FIG. 42), SN3/7f (FIG. 43), SN3/7g (FIG. 44), and SN3/7h (FIG. 45) was similar to or slightly better than that of the unmutated SN3/0.

### Example 7: Immunogenicity Comparison of Mutants of Recombinant Poliovirus Like Particles

Different mutants of poliovirus like particles types I, II, and III obtained in Example 4 were each used to immunize Wistar rats by intramuscular injection at an equivalent antigen protein content of 1 µg/dose, and meanwhile, the sabin inactivated poliovirus vaccine was set as a positive control. After 3 weeks, serum was collected to determine neutralizing antibody titers.

The sabin inactivated poliovirus vaccine: Poliovirus type I (sabin1), type II (sabin2), and type III (sabin3) were separately inoculated into Vero cells for culturing, and viruses were harvested, concentrated, purified, subjected to formaldehyde inactivation treatment, and mixed in proportion to prepare a trivalent inactivated vaccine. This product appeared as a clear and colorless liquid. The administration dose was: sabin1:sabin2:sabin3 = 15 DU/dose:45 DU/dose:45 DU/dose.

The detection results are shown in FIG. 47 and Table 7, and the results show that according to the immunogenicity evaluation, mutants m7a, m6a, m6c, m5, and m4 had better immunogenicity than the control sabin inactivated poliovirus vaccine (sIPV) and the unmutated group (SN1/0) at the same dose, and the average antibody titer of those mutants was significantly higher than that of the two groups described above. In addition, it was found that the VLP mutation combination containing the H1248P mutation site had significantly reduced immunogenicity.

**Table 7. Immunogenicity (log2) of mutants of recombinant poliovirus like particles type I**

| Sample code | Rat 1 | Rat 2 | Rat 3 | Rat 4 | Rat 5 | Rat 6 | Rat 7 | Rat 8 | Average |
|---|---|---|---|---|---|---|---|---|---|
| SN1/m8 | 6 | 2 | 6 | 2 | 2 | 2 | 2 | 2 | 3.0 |
| SN1/m7a | 6 | 4 | 10 | 6 | 10 | 9 | 5 | 4 | 6.8 |
| SN1/m7b | 2 | 2 | 6 | 2 | 3 | 8 | 2 | 2 | 3.4 |
| SN1/m7c | 2 | 4 | 2 | 3 | 2 | 3 | 4 | 2 | 2.8 |
| SN1/m6a | 6 | 6 | 10 | 9 | 10 | 9 | 7 | 8 | 8.1 |
| SN1/m6b | 4 | 2 | 2 | 2 | 2 | 2 | 2 | 2 | 2.3 |
| SN1/m6c | 5 | 11 | 11 | 2 | 2 | 9 | 7 | 10 | 7.1 |
| SN1/m5 | 10 | 6 | 10 | 10 | 7 | 4 | 8 | 11 | 8.3 |
| SN1/m5a | 2 | 6 | 7 | 2 | 2 | 3 | 4 | 6 | 4.0 |
| SN1/m4 | 6 | 8 | 6 | 11 | 10 | 11 | 8 | 7 | 8.4 |
| SN1/0 | 2 | 8 | 9 | 2 | 2 | 2 | 2 | 2 | 3.6 |
| sIPV | 7 | 2 | 4 | 6 | 7 | 4 | 4 | 7 | 5.1 |

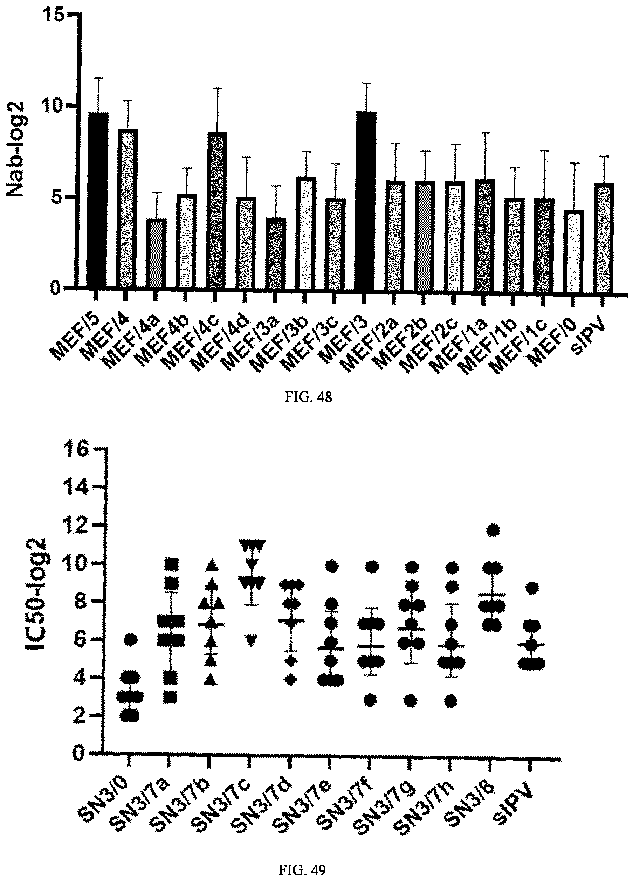

For different mutants of poliovirus like particles type II, mutants MEF/3, MEF/4c, MEF/4, and MEF/5 had the highest immunogenicity, followed by MEF/2b (see Table 8 and FIG. 48).

**Table 8. Immunogenicity (log2) of mutants of recombinant poliovirus like particles type II**

| Sample code | Rat 1 | Rat 2 | Rat 3 | Rat 4 | Rat 5 | Rat 6 | Rat 7 | Rat 8 | Average |
|---|---|---|---|---|---|---|---|---|---|
| MEF/5 | 8 | 7 | 12 | 10 | 9 | 8 | 12 | 11 | 10 |
| MEF/4 | 6 | 11 | 8 | 8 | 10 | 9 | 8 | 10 | 9 |
| MEF/4a | 4 | 3 | 5 | 2 | 6 | 5 | 4 | 2 | 4 |
| MEF4b | 4 | 8 | 6 | 4 | 4 | 5 | 6 | 5 | 5 |
| MEF/4c | 12 | 7 | 9 | 11 | 8 | 9 | 4 | 9 | 9 |
| MEF/4d | 4 | 8 | 7 | 6 | 3 | 2 | 4 | 7 | 5 |
| MEF/3a | 4 | 6 | 3 | 2 | 6 | 3 | 2 | 6 | 4 |
| MEF/3b | 6 | 8 | 7 | 5 | 4 | 6 | 8 | 6 | 6 |
| MEF/3c | 5 | 7 | 6 | 2 | 8 | 5 | 4 | 4 | 5 |
| MEF/3 | 11 | 9 | 11 | 9 | 10 | 7 | 12 | 10 | 10 |
| MEF/2a | 8 | 5 | 5 | 8 | 6 | 9 | 5 | 3 | 6 |
| MEF2b | 7 | 9 | 5 | 5 | 7 | 4 | 7 | 5 | 6 |
| MEF/2c | 5 | 4 | 6 | 7 | 9 | 7 | 8 | 3 | 6 |
| MEF/1a | 3 | 4 | 9 | 3 | 7 | 9 | 8 | 7 | 6 |
| MEF/1b | 4 | 5 | 3 | 8 | 6 | 5 | 4 | 7 | 5 |
| MEF/1c | 3 | 4 | 10 | 3 | 3 | 6 | 8 | 5 | 5 |
| MEF/0 | 9 | 3 | 2 | 7 | 2 | 5 | 3 | 6 | 5 |
| sIPV | 5 | 7 | 7 | 5 | 6 | 5 | 9 | 5 | 6 |

For different mutants of poliovirus like particles type III, all mutant strains were able to improve their immunogenicity to different degrees relative to the unmutated strain, with mutant SN3/8 having the highest immunogenicity (see Table 9 and FIG. 49).

**Table 9. Immunogenicity (log3) of mutants of recombinant poliovirus like particles type III**

| Sample code | Rat 1 | Rat 2 | Rat 3 | Rat 4 | Rat 5 | Rat 6 | Rat 7 | Rat 8 | Average |
|---|---|---|---|---|---|---|---|---|---|
| SN3/0 | 2 | 4 | 3 | 4 | 3 | 6 | 3 | 2 | 3 |
| SN3/7a | 6 | 3 | 10 | 4 | 9 | 7 | 7 | 6 | 7 |
| SN3/7b | 4 | 5 | 8 | 8 | 10 | 9 | 6 | 7 | 7 |
| SN3/7c | 10 | 9 | 11 | 11 | 6 | 9 | 11 | 9 | 10 |
| SN3/7d | 4 | 7 | 8 | 9 | 5 | 9 | 8 | 9 | 7 |
| SN3/7e | 4 | 8 | 4 | 6 | 10 | 5 | 4 | 7 | 6 |
| SN3/7f | 7 | 3 | 10 | 7 | 5 | 5 | 7 | 5 | 6 |
| SN3/7g | 3 | 8 | 6 | 9 | 6 | 10 | 7 | 8 | 7 |
| SN3/7h | 10 | 5 | 7 | 6 | 3 | 5 | 9 | 5 | 6 |
| SN3/8 | 10 | 8 | 7 | 8 | 7 | 10 | 8 | 12 | 9 |
| sIPV | 5 | 7 | 5 | 7 | 9 | 5 | 6 | 5 | 6 |

### Example 8: Immunogenicity Comparison of Mutants of Recombinant Poliovirus Like Particles

Different mutants of poliovirus like particles types I, II, and III obtained in Example 4 were each used to immunize Wister rats by intramuscular injection at an equivalent antigen protein content of 1 µg/dose, and meanwhile, the sabin inactivated poliovirus vaccine was set as a positive control. After 3 weeks, serum was collected to determine neutralizing antibody titers. Combinations of types I, II, and III are shown in Table 10, and immunogenicity results are shown in Table 11.

The sabin inactivated poliovirus vaccine: Poliovirus type I (sabin1), type II (sabin2), and type III (sabin3) were separately inoculated into Vero cells for culturing, and viruses were harvested, concentrated, purified, subjected to formaldehyde inactivation treatment, and mixed in proportion to prepare a trivalent inactivated vaccine. This product appeared as a clear and colorless liquid. The administration dose was: sabin1:sabin2:sabin3 = 15 DU/dose:45 DU/dose:45 DU/dose.

**Table 10. Combination design of mutants of recombinant poliovirus like particles**

| Group | I | II | III |
|---|---|---|---|
| A | SN1/m6a | MEF/3 | SN3/8 |
| B | SN1/m6c | | |
| C | SN1/m5 | | |
| D | SN1/m4 | | |
| E | Sabin inactivated poliovirus vaccine | | |

**Table 11. Immunogenicity of mutants of recombinant poliovirus like particles**

| Serotype | Sample code | Rat 1 | Rat 2 | Rat 3 | Rat 4 | Rat 5 | Rat 6 | Rat 7 | Rat 8 | Average |
|---|---|---|---|---|---|---|---|---|---|---|
| Type1 | A | 6 | 6 | 10 | 9 | 10 | 9 | 7 | 8 | 6 |
| | B | 9 | 6 | 11 | 9 | 7 | 4 | 7 | 11 | 9 |
| | C | 6 | 4 | 10 | 6 | 10 | 9 | 5 | 4 | 6 |
| | D | 2 | 6 | 7 | 5 | 7 | 3 | 4 | 6 | 2 |
| | E | 7 | 10 | 6 | 7 | 6 | 9 | 8 | 9 | 7 |
| Type2 | A | 9 | 4 | 10 | 7 | 5 | 6 | 9 | 8 | 9 |
| | B | 6 | 4 | 9 | 9 | 7 | 9 | 8 | 7 | 6 |
| | C | 6 | 5 | 3 | 9 | 6 | 5 | 8 | 7 | 6 |
| | D | 3 | 4 | 10 | 5 | 7 | 6 | 8 | 8 | 3 |
| | E | 4 | 5 | 3 | 9 | 5 | 4 | 8 | 2 | 4 |
| Type3 | A | 11 | 7 | 9 | 8 | 9 | 6 | 7 | 6 | 11 |
| | B | 2 | 7 | 10 | 4 | 9 | 7 | 8 | 6 | 2 |
| | C | 4 | 5 | 8 | 8 | 6 | 9 | 6 | 7 | 4 |
| | D | 5 | 7 | 8 | 6 | 9 | 8 | 9 | 9 | 5 |
| | E | 4 | 7 | 4 | 3 | 5 | 6 | 8 | 5 | 4 |

The results show that the immune effect of group A was the best, but the immune effects of groups B-D were all better than that of group E.

The above description is only for the purpose of illustrating the preferred examples of the present disclosure, and is not intended to limit the scope of the present disclosure. Any modifications, equivalents, and the like made without departing from the spirit and principle of the present disclosure shall fall in the claimed scope of the present disclosure.

The foregoing examples and methods described in the present disclosure may vary based on the abilities, experience, and preferences of those skilled in the art.

Certain sequence of the steps of methods listed in the present disclosure does not constitute any limitation on the sequence of steps of methods encompassed by the present disclosure.

## Claims

1. A recombinant *poliovirus* like particle (PVLP), wherein the PVLP is derived from:
(i) strain Sabin 1 of poliovirus type I;
(ii) strain MEF-1 of poliovirus type II; and/or
(iii) strain Sabin 3 of poliovirus type III.

2. The poliovirus like particle according to claim 1, wherein the PVLP is derived from poliovirus type I, and a structural protein of the PVLP comprises a mutation in at least one residue at a protomer interface, a VP2/VP3 interface, and/or a pentamer interface of the protein relative to a structural protein coded by the poliovirus type I.

3. The poliovirus like particle according to claim 2, wherein the structural protein of the PVLP further comprises a mutation in at least one residue at a binding pocket domain of the protein and/or an internal network of the protein.

4. The poliovirus like particle according to any one of claim 2 or 3, wherein the PVLP is derived from poliovirus type I, and the protein of the PVLP has the following mutations:
(i-1) at least a mutation at residue 3178 in the protomer interface of the protein;
(i-2) at least a mutation at residue 3119 in the VP2/VP3 interface of the protein; and/or
(i-3) at least one mutation at residues 2025 or 2057 in the pentamer interface of the protein.

5. The poliovirus like particle according to any one of claims 2-4, wherein the PVLP is derived from poliovirus type I, and the protein of the PVLP has the following mutations:
(i-1) at least a mutation at residue 3178 in the protomer interface of the protein;
(i-2) at least a mutation at residue 3119 in the VP2/VP3 interface of the protein; and/or
(i-3) at least one mutation at residues 2025 or 2057 in the pentamer interface of the protein; and
optionally, the protein of the PVLP further has the following mutations:
(i-4) at least one mutation at residues 1196 or 1134 in the binding pocket domain of the protein; and/or
(i-5) at least a mutation at residue 4018 in the internal network of the protein.

6. The poliovirus like particle according to any one of claims 2-5, wherein the PVLP is derived from poliovirus type I, and the protein of the PVLP has the following mutations:
(i-1) at least residue mutation Q3178L in the protomer interface of the protein;
(i-2) at least residue mutation L3119M in the VP2/VP3 interface of the protein; and/or
(i-3) at least one of residue mutations T2025A or D2057E in the pentamer interface of the protein.

7. The poliovirus like particle according to any one of claims 2-6, wherein the PVLP is derived from poliovirus type I, and the protein of the PVLP has the following mutations:
(i-1) at least residue mutation Q3178L in the protomer interface of the protein;
(i-2) at least residue mutation L3119M in the VP2/VP3 interface of the protein; and/or
(i-3) at least one of residue mutations T2025A or D2057E in the pentamer interface of the protein;
optionally, the protein of the PVLP further has the following mutations:
(i-4) at least one of residue mutations V1196L or F1134L in the pocket domain of the protein; and/or
(i-5) at least residue mutation R4018G in the internal network of the protein.

8. The poliovirus like particle according to any one of claims 1-7, wherein the PVLP is derived from poliovirus type I, and the protein of the PVLP has the following mutations:
i-a) one or more of T2025A, D2057E, L3119M, Q3178L, and R4018G;
and optionally,
i-b) one or more mutations of H1248P, F1134L, and V1196L.

9. The poliovirus like particle according to any one of claims 1-8, wherein the PVLP is derived from poliovirus type I, and the protein of the PVLP has the following mutations:
i-A) T2025A, D2057E, L3119M, Q3178L, H1248P, F1134L, V1196L, and R4018G; or
i-B) T2025A, D2057E, L3119M, Q3178L, F1134L, V1196L, and R4018G; or
i-C) T2025A, D2057E, L3119M, Q3178L, H1248P, V1196L, and R4018G; or
i-D) T2025A, D2057E, L3119M, Q3178L, H1248P, F1134L, and R4018G; or
i-E) T2025A, D2057E, L3119M, Q3178L, V1196L, and R4018G; or
i-F) T2025A, D2057E, L3119M, Q3178L, H1248P, and R4018G; or
i-G) T2025A, D2057E, L3119M, Q3178L, and H1248P; or
i-H) T2025A, D2057E, L3119M, Q3178L, F1134L, and R4018G; or
i-I) T2025A, D2057E, L3119M, Q3178L, and R4018G; or
i-J) T2025A, D2057E, L3119M, and Q3178L.

10. The poliovirus like particle according to any one of claims 1-9, wherein the PVLP is derived from poliovirus type I, and residue 1248 is not mutated in the protein of the PVLP.

11. The poliovirus like particle according to any one of claims 1-10, wherein the PVLP is derived from poliovirus type II, and the protein of the PVLP has the following mutations:
(ii-1) at least a mutation at residue 3178 in the protomer interface of the protein;
(ii-2) at least a mutation at residue 1041 in the pentamer interface of the protein; and/or
(ii-3) at least a mutation at residue 1159 in the pocket domain of the protein.

12. The poliovirus like particle according to claims 1-11, wherein the PVLP is derived from poliovirus type II, and the protein of the PVLP has the following mutations:
(ii-1) at least residue mutation Q3178L in the protomer interface;
(ii-2) at least residue mutation T1041I in the pentamer interface; and/or
(ii-3) at least residue mutation Y1159F in the pocket domain.

13. The poliovirus like particle according to claims 1-12, wherein the PVLP is derived from poliovirus type III, and the protein of the PVLP has the following mutations:
(iii-1) at least one mutation at residues 2215 or 3091 in the protomer interface of the protein;
(iii-2) at least one mutation at residues 2018 or 3085 in the pentamer interface of the protein;
(iii-3) at least a mutation at residue 1132 in the pocket domain of the protein;
(iii-4) at least a mutation at residue 2241 in the VP2/VP3 interface of the protein; and/or (iii-5) at least a mutation at residue 3019 in the internal network of the protein.

14. The poliovirus like particle according to claims 1-13, wherein the PVLP is derived from poliovirus type III, and the protein of the PVLP has the following mutations:
(iii-1) at least one of residue mutations L2215M or F3091S in the protomer interface of the protein;
(iii-2) at least one of residue mutations L2018I or L3085F in the pentamer interface of the protein;
(iii-3) at least residue mutation F1132L in the pocket domain of the protein;
(iii-4) at least residue mutation D2241E in the VP2/VP3 interface of the protein; and/or
(iii-5) at least residue mutation H3019Y in the internal network of the protein.

15. A gene encoding the poliovirus like particle according to any one of claims 1-14, wherein preferably, the gene comprises genes VP1, VP0, and VP3 encoding proteins VP1, VP0, and VP3, wherein the proteins VP1, VP0, and VP3 have mutations as defined for the poliovirus like particle according to any one of claims 1-12.

16. A method for preparing the poliovirus like particle according to any one of claims 1-15, wherein the preparation method comprises amplification to obtain the gene according to claim 15 and construction of a vector carrying the gene described above; preferably, the preparation method comprises linking in tandem of any two structural genes of genes VP1, VP0, and VP3, inserting them into one expression cassette of the vector, and inserting another gene into a second expression cassette of the vector, thereby obtaining an expression vector comprising different mutant genes of the poliovirus like particle; more preferably, the preparation method comprises inserting the genes into a pBacPAK8-Duet plasmid, wherein two structural genes are linked in tandem via IntP2A and then inserted into one expression cassette of the pBacPAK8-Duet plasmid, and another gene is inserted into a second expression cassette of the pBacPAK8-Duet plasmid, thereby obtaining a vector comprising different mutant genes of the poliovirus like particle.

17. The preparation method according to claim 16, wherein a host cell of the vector comprises a mammalian cell (e.g., an HEK293 cell or a CHO cell), a yeast cell, or an insect cell (e.g., *Spodoptera frugiperda* cell sf9 or *Trichoplusia ni* cell Tn); preferably, the host cell is *Spodoptera frugiperda* cell Sf9.

18. The preparation method according to any one of claims 16-17, wherein the preparation method further comprises co-transfecting insect cell sf9 with the vector and a baculovirus genome in a *flash* BAC system and obtaining a recombinant baculovirus capable of expressing different mutants of the poliovirus like particle through homologous recombination.

19. An expression vector system, wherein the expression vector system carries the gene according to claim 15; preferably, the gene comprises genes VP1, VP0, and VP3; more preferably, any two structural genes of the genes VP1, VP0, and VP3 are linked in tandem and then inserted into a first expression cassette of the expression vector, and another gene is inserted into a second expression cassette of the expression vector, thereby obtaining an expression vector comprising different mutant genes of the poliovirus like particle; further preferably, the two structural genes are linked in tandem via IntP2A.

20. Use of the poliovirus like particle according to any one of claims 1-14 in preparing a medicament for preventing or treating poliomyelitis, wherein preferably, the medicament is a vaccine.

21. A vaccine, comprising the recombinant poliovirus like particle according to any one of claims 1-14, wherein preferably, the vaccine further comprises an adjuvant; more preferably, the adjuvant is any one or more of an oil adjuvant, an oil-in-water adjuvant, a water-in-oil adjuvant, a water-in-oil-in-water adjuvant, an aluminum salt, and a polysaccharide adjuvant; preferably, the vaccine comprises 1-300 DU/dose of the recombinant poliovirus like particle per unit dose, and a content of the adjuvant is 0.01-0.8 mg/dose; the vaccine further comprises a pharmaceutically acceptable auxiliary material; more preferably, the auxiliary material comprises an osmotic pressure regulator and a surfactant; more preferably, a dosage form of the vaccine is any one or more of a liquid formulation, a powder, an injection, and a tablet; further preferably, the vaccine is a subcutaneous injection, an intramuscular injection, or a microinjection.
